# EUROPÄISCHE PATENTSCHRIFT

(11) **EP 3 362 008 B1**
(45) Veröffentlichungstag und Bekanntmachung des Hinweises auf die Patenterteilung: **14.08.2019**
(21) Anmeldenummer: 17764782.3
(22) Anmeldetag: 31.08.2017
(51) Int. Cl.: A61F 5/01

(54) **KIPPBARE ORTHESENGELENKSCHIENE**
TILTABLE ARTICULATED JOINT BRACE
LANGUETTE D'ARTICULATION ORTHÉTIQUE INCLINABLE

(30) Priorität: 06.09.2016 DE 102016216862
(43) Veröffentlichungstag der Anmeldung: 22.08.2018
(73) Patentinhaber: Bauerfeind AG, 07937 Zeulenroda-Triebes (DE)
(72) Erfinder: BAUERFEIND, Hans B., 07937 Zeulenroda-Triebes (DE); PANZER, Dominique, 07937 Zeulenroda-Triebes (DE)
(74) Vertreter: Schwahn, Hartmut
(86) Internationale Anmeldenummer: PCT/EP2017/071850
(87) Internationale Veröffentlichungsnummer: WO 2018/046382

(56) Entgegenhaltungen:
- US-A- 621 366
- US-A- 5 542 774
- US-A- 5 658 243
- US-A1- 2005 187 506
- US-A1- 2009 216 164
- US-B1- 8 858 480

## Beschreibung

Die Erfindung betrifft eine verbesserte Gelenkorthese zur Stützung oder Korrektur eines Körper- oder Extremitätengelenks, spezifisch eine Gelenkschiene mit einstellbarer Verkippung der Gelenkschenkel.

Orthesen sind medizinische Hilfsmittel, welche in der Orthopädie zur Aufrechterhaltung, Stabilisierung oder Wiederherstellung einer Gelenkfunktion dienen können. Zur therapeutischen oder prophylaktischen mechanischen Unterstützung der Gelenkfunktion, besonders auch zur kontrollierten Führung der Gelenkbewegung, werden insbesondere derartige Gelenkorthesen eingesetzt, die zumindest eine mechanisch stabile Gelenkschiene aufweisen. Diese Gelenkschienen dienen der mechanischen Entlastung und Unterstützung des Körpergelenks und auch besonders der gezielten Korrektur der Gelenkstellung; sie sollen der Beuge- oder Streckbewegung des zu stützenden Gelenks möglichst genau folgen.

Bekannte Gelenkschienen weisen zwei flache, aneinander gelagerte und zueinander verschwenkbare Gelenkschenkel auf, die sich im angelegten Zustand der Orthese jeweils diesseits und jenseits des zu stützenden Körpergelenks erstrecken und dieses somit überbrücken. Zur Stützung und Korrektur der Gelenkbewegung eines Körpergelenks hat es sich inzwischen als vorteilhaft herausgestellt, dass die Gelenkschenkel aus ihrer primären Schwenkebene heraus gegeneinander verkippbar sind. Eine derartige Gelenkschiene ist zum Beispiel in der DE 10 2008 009 273 A1 beschrieben. Nachteilig bei dieser Bauform ist aber besonders, dass die Verkippung der Gelenkschenkel gegeneinander limitiert ist, Torsion auftreten kann und in der Praxis meist nur zwischen zwei Kippstellungen gewechselt werden kann. Außerdem ist der mechanische Aufbau sperrig und führt zu räumlich ausladenden Orthesen-Konstruktionen. Es lag der vorliegenden Erfindung das technische Problem zugrunde, eine Gelenkschiene mit gegeneinander verkippbaren Gelenkschenkeln so weiterzubilden, dass die Verkippung stufenlos und über einen großen Kippwinkelbereich einstellbar ist, wobei insbesondere eine flache und kompakte Bauform und hohe mechanische Stabilität erreicht werden soll. Besonderes soll die Verkippung mit einfachen Handgriffen von außen auch im angelegten Zustand der Gelenkorthese einstellbar und sicher festlegbar sein.

Das technische Problem wird vollständig gelöst durch die Gelenkschienenanordnung nach Anspruch 1, besonders eine Gelenkschiene mit zwei gattungsgemäß in einer insbesondere monoaxialen Lageranordnung gegeneinander verschwenkbaren Gelenkschenkeln, wobei der erste Gelenkschenkel an dem zweiten Gelenkschenkel schwenkbar gelagert ist, wobei erfindungsgemäß insbesondere der Kopf des ersten (oberen) Gelenkschenkels die Lageranordnung fasst oder trägt und der Kopf des zweiten (unteren) Gelenkschenkels mit dieser Lageranordnung mechanisch, das heißt insbesondere kraft- und/oder formschlüssig verbindbar oder verbunden ist, um das Schwenkgelenk der beiden Gelenkschenkel der Gelenkschiene zu bilden.

Erfindungsgemäß ist die Gelenkschiene dadurch gekennzeichnet, dass der Kopf des ersten Gelenkschenkels, insbesondere aber die Lageranordnung an dem Kopf des ersten Gelenkschenkels selbst, zu dem Kopf des zweiten Gelenkschenkels hin eine gekrümmte Gleitfläche aufweist, die ein Abschnitt eines Zylindermantels ist, also eine um eine einzige Achse gekrümmte Fläche, oder alternativ kalottenförmig ist. Dabei weist der Kopf des zweiten Gelenkschenkels eine entsprechende zylindrische Krümmung oder kalottenförmige Wölbung auf. Der gekrümmte oder gewölbte Abschnitt des Kopfs des zweiten Gelenkschenkels liegt auf oder in der zylindrischen oder kalottenförmigen Gleitfläche des Kopfs des ersten Gelenkschenkels und ist dort spielfrei gelagert. Der Kopf ist dort formschlüssig in der Art eines Axiallagers beziehungsweise in der Art eines Kugelkopfgelenks geführt. Dadurch wird ein Kippgelenk gebildet. Dort ist der zweite Gelenkschenkel gegenüber dem ersten Gelenkschenkel, zur Schwenkachse des Schwenkgelenks hin, das heißt aus der primären Schwenkebene der Gelenkschiene heraus gegen den ersten Gelenkschenkel verkippbar. Erfindungsgemäß weist der Kopf des zweiten Gelenkschenkels ein Fenster auf, worin ein Klemmstein geführt ist, der an die Lageranordnung des ersten Gelenkschenkels spannbar ist, um den Kopf des zweiten Gelenkschenkels in der gekrümmten Gleitfläche kraftschlüssig zu fixieren, und der Klemmstein weist Klemmschultern auf, welche die Rückseite des Kopfes hintergreifen, um den Kopf an die Lageranordnung des ersten Gelenkschenkels zu klemmen.

Durch die Verklemmung des Kopfs des zweiten Gelenkschenkels an der Gleitfläche der ersten Gelenkschenkels kann an dem Kippgelenk der jeweilige Verkippungswinkel frei eingestellt und schließlich festgelegt werden. Die Verschwenkung der beiden Gelenkschenkel in dem durch die Lageranordnung gebildeten Schenkgelenk bleibt davon unberührt.

Die Erfindung erlaubt so die Bereitstellung einer Gelenkschiene mit gattungsgemäß zueinander verschwenkbaren Gelenkschenkeln und damit auch einer verbesserten Orthese mit dieser Gelenkschiene, worin eine zusätzliche Verkippung oder Torsion der beiden zueinander verschwenkbaren Gelenkschenkel stufenlos und über einen großen Kippwinkelbereich einstellbar ist, wobei eine flache und kompakte Bauform der gesamten Gelenkschiene und eine hohe mechanische Stabilität ohne Verspannungs- oder Verklemmungsneigung erreicht ist. Dabei kann die Verkippung mit einfachen Handgriffen von außen auch im angelegten Zustand der Gelenkorthese eingestellt und sicher festgelegt werden.

Bevorzugt weist der Kopf des ersten Gelenkschenkels zu dem Kopf des zweiten Gelenkschenkels hin eine konkav gekrümmte Gleitfläche auf, die entweder ein Abschnitt eines Zylindermantels bildet oder alternativ kalottenförmig ist. Dabei weist der Kopf des zweiten Gelenkschenkels eine entsprechende zylindrische oder kalottenförmige konvexe Wölbung, das heißt konvexe Krümmung auf. Die Wölbung des Kopfs des zweiten Gelenkschenkels liegt in der konkav zylindrischen oder konkav kalottenförmigen Gleitfläche an dem Kopf des ersten Gelenkschenkels und ist dort spielfrei gelagert.

In einer dazu alternativen Ausgestaltung weist der Kopf des ersten Gelenkschenkels zu dem Kopf des zweiten Gelenkschenkels hin eine konvex gekrümmte Gleitfläche auf, die entweder ein Abschnitt eines Zylindermantels bildet oder alternativ kalottenförmig ist. Dabei weist der Kopf des zweiten Gelenkschenkels eine entsprechende zylindrische oder kalottenförmige konkave Krümmung auf. Die Krümmung des Kopfs des zweiten Gelenkschenkels liegt auf der konvex zylindrisch oder konvex kalottenförmig gewölbten Gleitfläche an dem Kopf des ersten Gelenkschenkels und ist dort spielfrei gelagert.

Die Konstruktion sieht in einer ersten spezifischen Ausführung vor, dass durch das formschlüssige Zusammenwirken von zylindrisch gekrümmter Gleitfläche an dem Kopf des ersten Gelenkschenkels und dem zylindrisch gekrümmten Kopf des zweiten Gelenkschenkels ein Abschnitt eines Axiallagers gebildet wird, wobei der Kopf des ersten Gelenkschenkels den zylindrischen Lagerschalenabschnitt und der Kopf des zweiten Gelenkschenkels den zylindrischen Lagerzapfenabschnitt bildet, der auf in dem Lagerschalenabschnitt läuft und insbesondere dort festlegbar, besonders klemmbar ist. Dabei ist bevorzugt, dass das so gebildete monoaxiale Kipplager eine Lagerachse besitzt, die in der Schwenkebene der Lageranordnung oder parallel dazu, alternativ bevorzugt in einem Winkel von -20° bis +20° zur Schwenkebene der Gelenkschiene verläuft, sodass neben der festlegbaren Verkippung eine konkrete Torsion der Gelenkschienen gegeneinander gewählt realisiert ist.

Die Konstruktion sieht in einer zweiten spezifischen Ausführung vor, dass durch das formschlüssige Zusammenwirken von kalottenförmig gekrümmter Gleitfläche an dem Kopf des ersten Gelenkschenkels und dem kalottenförmig gekrümmten Kopf des zweiten Gelenkschenkels ein Abschnitt eines Kugelgelenks gebildet ist, was dort festlegbar, besonders klemmbar ist. Dabei ist bevorzugt, dass das Kugelgelenkslager nicht nur die Verkippung in einer Kippachse ermöglicht, sondern neben der Verkippung gegen die primäre Schwenkebene oder alternativ zu dieser Verkippung eine Torsion der beiden Gelenkschienen gewählt werden kann. Dadurch ist in Einzelfällen eine besonders genaue Anpassung und Führung der Gelenkbewegung des zu stützenden oder zu führenden Körpergelenks möglich. Der Kippwinkel zur primären Schwenkebene des Kugelgelenks beträgt von -20° bis +20°, der alternative oder zusätzliche Torsionswinkel der beiden Gelenkschienen gegeneinander beträgt bevorzugt von -20° bis +20°.

In bevorzugter Ausgestaltung bildet der Kopf des ersten Gelenkschenkels einen Ring aus, worin die Lageranordnung zur Ausbildung eines Axiallagers gefasst und insbesondere direkt eingesetzt ist. Über diese Lageranordnung ist der Kopf des zweiten Gelenkschenkels mit dem Kopf des ersten Gelenkschenkels verbunden.

In einer bevorzugten Ausführung ist die Lageranordnung an dem Kopf des ersten Gelenkschenkels durch eine spezielle Profilscheibe zu dem zweiten Gelenkschenkel hin abgeschlossen, wobei die Profilscheibe auf der zum zweiten Gelenkschenkel hin weisenden Seite die erfindungsgemäße konkave Gleitfläche aufweist.

Um den Kopf der zweiten Gelenkschiene an der gekrümmten Gleitfläche der Lageranordnung des ersten Gelenkschenkels zu führen, dort zu halten und zu fixieren, ist vorgesehen, dass in dem Kopf der zweiten Gelenkschiene eine Längsnut, ein Fenster oder Langloch ausgebildet ist, worin ein Klemmstein durchtritt oder dort gleitend geführt ist. Dieser Klemmstein ist an den Kopf des ersten Gelenkschenkels spannbar, um den Kopf des zweiten Gelenkschenkels gegen die konvexe Gleitfläche des ersten Gelenkschenkels zu pressen. Durch den erzielten Form- und Reibschluss wird der Verkippwinkel festgelegt. Dabei ist erfindungsgemäß vorgesehen, dass der Klemmstein untere Klemmabschnitte oder Klemmarme, das heißt Klemmschultern, aufweist, welche die Unterseite des Kopfes der zweiten Gelenkschiene hintergreifen, um den Kopf an die Lageranordnung und den Kopf des ersten Gelenkschenkels zu ziehen und dort festzuklemmen. Bevorzugt sind dabei die Klemmabschnitte, das heißt Klemmschultern, des Klemmsteins und bevorzugt zusätzlich die Unterseite des Kopfes der zweiten Gelenkschiene, die mit diesen Klemmabschnitten in Kontakt kommen, mit einer rauen oder gezahnten Oberflächenstruktur versehen, um die Klemmung und Fixierung durch zu Reib- und Formschluss verbessern.

Zum Spannen des Klemmsteins zur Klemmung und Fixierung der zweiten Gelenkschiene am Lager der ersten Gelenkschiene weist dieser besonders eine durchgehende Bohrung oder bevorzugt ein Innengewinde auf, wo ein Achsbolzen der Lageranordnung durchsteckbar und bevorzugt einschraubbar ist. Dadurch kann der Klemmstein gegen den Kopf des ersten Gelenkschenkels gezogen werden. Dabei ist bevorzugt vorgesehen, dass dieser Achsbolzen auch das zentrale Element der Lageranordnung ist, und in Verbindung mit diesem Klemmstein die Lageranordnung zusammenhält, die Lageranordnung in dem Kopf des ersten Gelenkschenkels hält.

Besonders durch die Betätigung des zentralen Achsbolzens, der bevorzugt als Schraube mit Schraubenkopf oder mit einem Exzenterklemmsystem ausgestaltet ist, kann die Verstellung des Verkippwinkels auch im angelegten Zustand der Gelenkorthese "von außen" und zentral erfolgen. In einer ersten Variante ist der zentrale Achsbolzen mit einem an sich bekannten Exzenterspannhebel ausgestattet, wie dieser beispielsweise zum Einspannen von Laufrädern in Gabel- oder Rahmenauge eines Fahrrads bekannt ist. In einer alternativen Variante ist der Achsbolzen eine Zylinderschraube mit Schraubenkopf, die "von außen" angezogen werden kann. So kann der Träger der Gelenkorthese auch im angelegten Zustand eine Verstellung der Verkippung der Gelenkschiene, je nach Bewegungszustand oder Therapieanforderung, leicht selbst vornehmen. In einer bevorzugten Variante ist der zentrale Achsbolzen, sein Verstellmechanismus oder Schraubenkopf nach außen durch einen auf die Gelenkschiene aufgesetzten Lagerdeckel, der bevorzugt in den Kopf des ersten Gelenkschenkels einrastet, mechanisch und vor Manipulation geschützt.

Durch die erfindungsgemäße Ineinanderschachtelung der einzelnen Bauelemente wird zum Einen die mechanische Verkopplung der Elemente zueinander verbessert, zum anderen wird die gesamte "Bauhöhe" der Anordnung reduziert, sodass vorteilhafterweise das Gesamtgelenk flach gehalten werden kann und an der Orthese wenig aufbaut. In einer spezifischen Ausgestaltung ist dazu außerdem vorgesehen, dass der Klemmstein einen zylindrischen Hals aufweist, der unmittelbar in die Lageranordnung eingreift und zur Kompaktheit der Bauform bei gleichzeitig hoher mechanischer Stabilität der Anordnung beiträgt.

Bevorzugt ist in der Ausführung mit konkaven zylindrischer Gleitfläche der Lageranordnung, insbesondere der Profilscheibe, dort eine zusätzliche Ausnehmung ausgebildet, insbesondere eingefräst, worin der eckige Körper des Klemmsteins formschlüssig eingreifen kann, um im Zusammenwirken mit der Nut des Kopfes der zweiten Gelenkschiene, worin der Körper des Klemmsteins ebenfalls formschlüssig geführt wird, eine Verdrehsicherung zu bilden, um den zylindrisch gekrümmten Kopf der zweiten Gelenkschiene an der zylindrischen Gleitfläche der Lageranordnung gegen Verdrehen zu sichern. Damit wird die Funktion des monoaxialen Kipplagers an der zylindrischen Gleitfläche gesichert.

Bevorzugt ist in der Lageranordnung, besonders in der Profilscheibe, welche das Lager abschließt, zumindest ein Langloch oder eine Kulisse in Form einer Nut oder Ausnehmung vorgesehen, worin mindestens ein Limitierungsstift eingreift, der in dem Kopf des ersten Gelenkschenkels in einer Aufnahme geführt ist. Dadurch kann die Verschwenkung des Lagers an dem ersten Gelenkschenkel - und damit die Verschwenkung des zweiten Gelenkschenkels gegenüber dem ersten Gelenkschenkel - limitiert werden. Vorzugsweise weist der Kopf des ersten Gelenkschenkels dabei eine oder mehrere Aufnahmen in Form von Bohrungen auf, worin wahlweise Limitierungsstifte eingesetzt oder eingerastet werden können. In bevorzugter Ausgestaltung werden die Limitierungsstifte durch einen Deckel, welcher auf den Kopf des ersten Gelenkschenkels aufgesteckt wird, gegen ein Herausgleiten aus den Bohrungen geschützt. Vorteilhafterweise sind die Limitierungsstifte von außen auch bei angelegter Gelenkorthese auswechselbar und damit die Schwenklimitierung, je nach Bewegungsanforderung und/oder Therapieansatz, einstellbar.

Gegenstand der Erfindung ist auch eine Gelenkorthese, welche zumindest eine der hierin beschriebenen Gelenkschienen enthält. Ein spezifischer Gegenstand ist eine Hüftgelenksorthese zur Korrektur und/oder Stützung der Hüftgelenksbewegung. Ein anderer spezifischer Gegenstand ist eine Kniegelenksorthese zur Stützung und/oder Stützung des Kniegelenks. Ein weiterer spezifischer Gegenstand ist eine Sprunggelenksorthese. Weitere spezifische Gegenstände sind eine Orthese zur Korrektur und/oder Stützung des Ellbogengelenks, Gelenkschienen zur Stützung von Fingergelenken, Zehengelenken und/oder Zehengrundgelenken.

Die Erfindung wird anhand der Figuren näher beschrieben, ohne dass die dort jeweils dargestellten spezifischen Ausführungsbeispiele beschränkend zu verstehen wären.

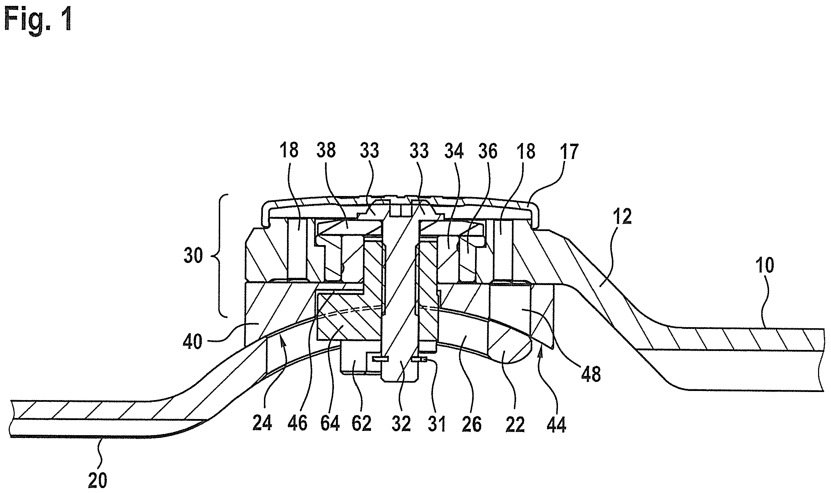

Figur 1 zeigt einen schematischen Querschnitt durch den Gelenkbereich einer ersten Ausführung der erfindungsgemäßen Gelenkschiene. Der Kopf 12 des ersten Gelenkschenkels 10 ist gekröpft und ringförmig ausgebildet. Er trägt die Elemente der Lageranordnung 30, und zwar im Einzelnen einen zentralen Achsbolzen 32, welcher über seinen Kopf 33 die obere Lagerabschlussscheibe 38 hält, welche das Lager zur Gelenkaußenseite hin abschließt. Zur Gelenkinnenseite hin wird das Lager hier durch die Profilscheibe 40 abgeschlossen, welche zu der oberen Abschlussscheibe 38 durch den Abstandsring 34 beabstandet wird. Die Außenschulter 14 des Ringlagers ist in dem Kopf 12 des ersten Gelenkschenkels ausgebildet. Zwischen dem rotierenden Innenteil der Lageranordnung 30 mit den Komponenten 32, 38, 34 und 40 ist ein Gleitring 36 angeordnet, welcher eine Schulter aufweist, die auf der Schulter 14 aufliegt, um das Lager in axialer und radialer Richtung zu sichern. Erfindungsgemäß wird die Lageranordnung 30 zusammengehalten, indem der Achsbolzen 32 in den Klemmstein 60 eingreift und dort insbesondere verschraubbar ist und diesen gegen die Profilscheibe 40 spannt. In der dargestellten Ausgestaltung wird der Klemmstein nach unten hin durch einen Sprengring 31 an dem Achsbolzen 32 gesichert, sodass die Anordnung nach Lockern des Achsbolzens 32 an den Klemmstein 60 nicht auseinander fällt. Der zylindrische Hals 66 des Klemmsteins taucht in die Lageranordnung 30 ein und ist dort an dem Abstandsring 38 spielfrei geführt. Der Klemmstein läuft unterhalb der Lageranordnung 30 in dem Fenster 26, das in dem Kopf 22 des unteren Gelenkschenkels 20 ausgebildet ist. Der Klemmstein 60 sitzt mit seinem eckigen Körper 64 gleichzeitig in einer Ausnehmung 46 der Profilscheibe 40 und sichert dadurch den Kopf 22 des unteren Gelenkschenkels 20 gegen Verdrehen gegen die Profilscheibe 40. Um den Kopf 22 an der konkaven Gleitfläche 44 der Profilscheibe 40 zu fixieren, hintergreift der Klemmstein 60 mit unteren Klemmabschnitten 62 den Kopf 22 auf dessen Unterseite. Wird der Klemmstein 60 mittels Achsbolzen 32 gespannt, wird der gewölbte Kopf 22 des unteren Gelenkschenkels 20 gegen die Profilscheibe 40 gedrückt, was gleichzeitig die Lageranordnung 30 zusammenhält und gleichzeitig den Verkippwinkel des unteren Gelenkschenkels 20 gegenüber dem oberen Gelenkschenkel 10 an der konvexen Gleitfläche 44 durch Reib- und Formschluss festlegt.
   Eine bogenförmige Kulisse 48 in der Profilscheibe 40 ist optional vorgesehen. Limitierungsstifte 70 (nicht gezeigt), welche in einer oder mehreren Bohrungen 18 im Kopf 12 der oberen Gelenkschiene 10 geführt oder fixiert sind, ragen, in die Kulisse 48 der Profilscheibe 40 und schlagen bei Verschwenkung der beiden Gelenkschenkel 10, 20 an der Wand der Kulisse 48 an, um den Verschwenkwinkel optional zu limitieren. Die Limitierungsstifte sind herausnehmbar, um die Limitierung aufzuheben. Ein Lagerdeckel 17, welcher auf den Kopf 12 gesetzt wird, verhindert insbesondere das Herausrutschen der Limitierungsstifte 70 aus den Bohrungen 18.

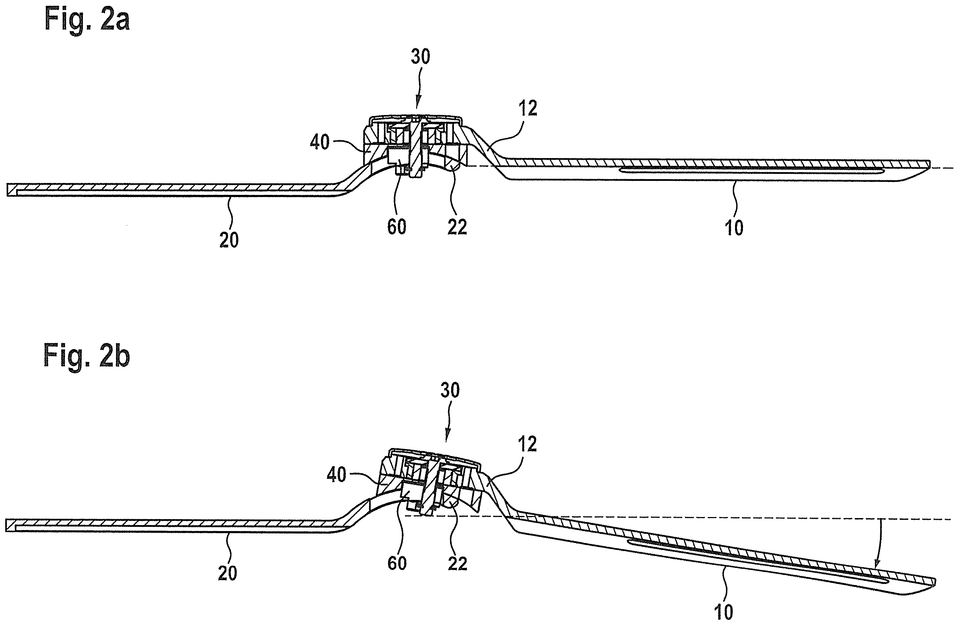

Figuren 2a und 2b zeigen jeweils im schematischen Querschnitt die Komponenten der Gelenkschiene nach Figur 1 bei unterschiedlichen Verkippungen der Gelenkschenkel 10, 20 zueinander. Figur 2a zeigt eine gestreckte, nicht gekippte Ausrichtung, wobei beide Gelenkschenkel 10, 20 innerhalb der primären Schwenkebene schwenken; Figur 2b zeigt eine Ausrichtung, bei der der untere Gelenkschenkel gegenüber dem oberen Gelenkschenkel verkippt ist. Dazu gleitet der untere Gelenkschenkel an seinem Kopf 22 in der konkaven Gleitfläche der Profilscheibe 40 und wird durch den Klemmstein 60 an dem oberen Gelenkschenkel 10 gehalten.

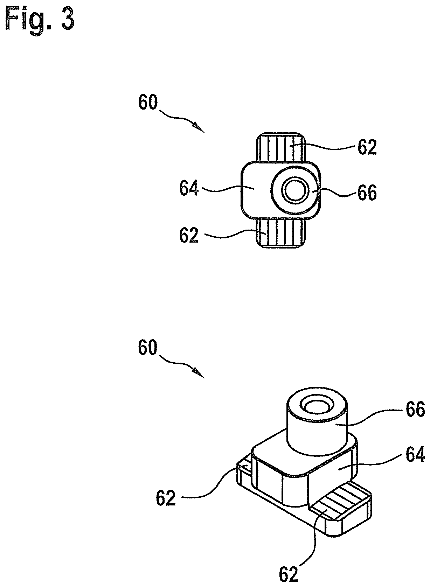

Figur 3 zeigt eine Draufsicht und eine Schrägansicht einer bevorzugten Ausgestaltung des Klemmsteins 60 mit einem oberen zylindrischen Hals 66, welcher in die Lageranordnung 30 eingreifen kann, einem eckigen Körper 64, der in dem Fenster 26 des Kopfes 22 des unteren Gelenkschenkels 20 läuft und bevorzugt dort geführt werden kann und bevorzugt gleichzeitig in einer entsprechenden Ausnehmung 46 der Profilscheibe 40 sitzen kann. Die sich beidseitig erstreckenden unteren Klemmabschnitte 62 weisen bevorzugt an ihrer zum Kopf 22 gewandten Seite eine raue oder gezahnte Oberfläche auf, um dort die reib- und formschlüssige Haftung zu verbessern.

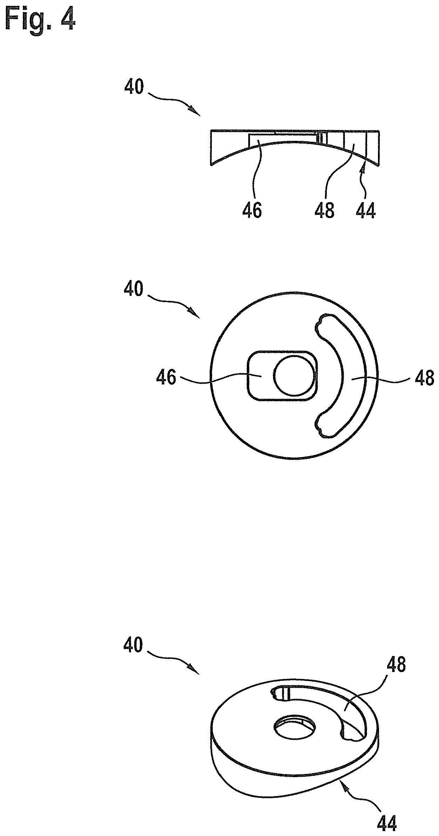

Figur 4 zeigt schematische Ansichten der Profilscheibe 40 mit einer unteren zylindrischen konkaven Ausnehmung 44, woran ein zylindrisch gewölbter Kopf 22 der unteren Gelenkschiene 20 gleiten kann. Die Profilscheibe 40 weist hier zusätzlich zu der zentralen Bohrung eine Ausnehmung 46 auf, worin ein eckiger Klemmstein 60 formschlüssig eingreifen kann und ein Verdrehen der Komponenten zueinander verhindern kann. Bevorzugt ist zusätzlich eine Kulisse 48 vorgesehen, worin ein oder mehrere Limitierungsstifte 70, die in dem Kopf 12 des oberen Gelenkschenkels 10 fixiert sind, ragen können, um eine Schwenklimitierung zu bilden.

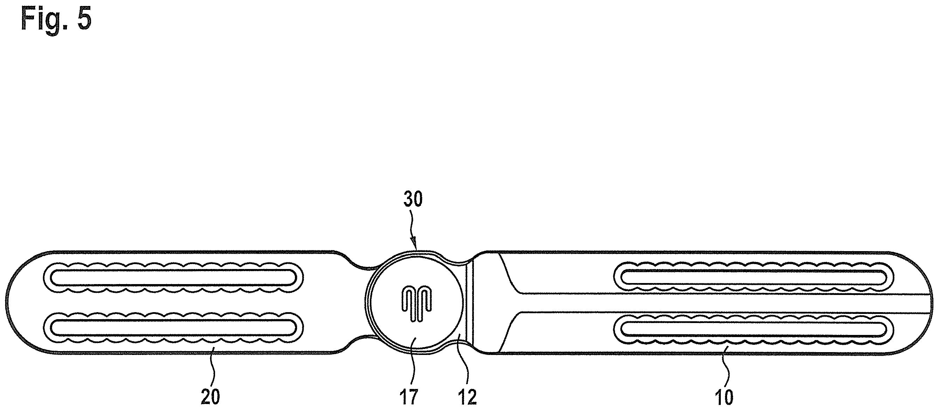

Figur 5 zeigt eine Draufsicht einer gebrauchsfertigen typischen Ausführung der erfindungsgemäßen Gelenkschiene mit unterem Gelenkschenkel 20, der über die Lageranordnung 30, welche mit Deckel 17 abgedeckt ist, mit dem oberen Gelenkschenkel 10 gelenkig verbunden ist.

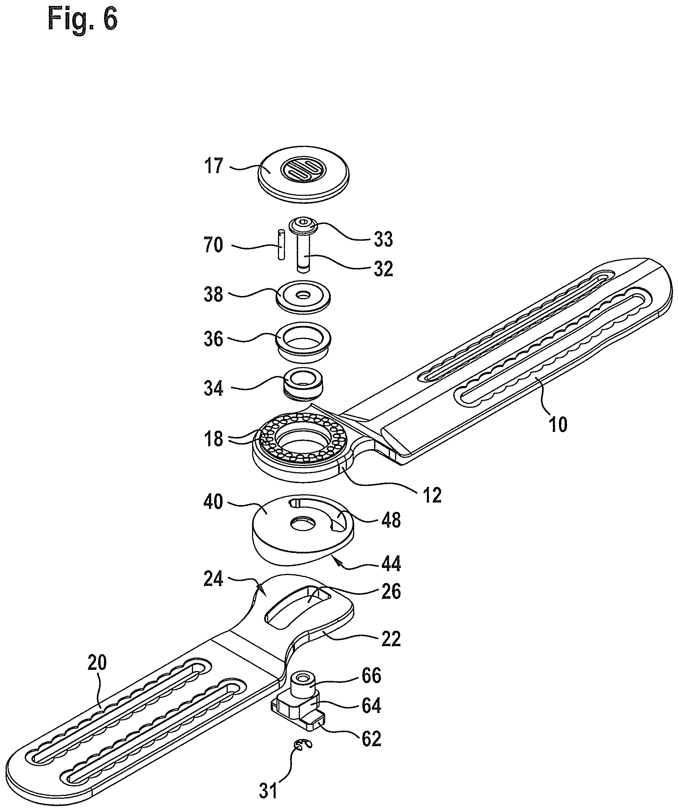

Figur 6 zeigt eine Explosionsdarstellung der spezifischen Ausgestaltung nach den Figuren 1 bis 5.

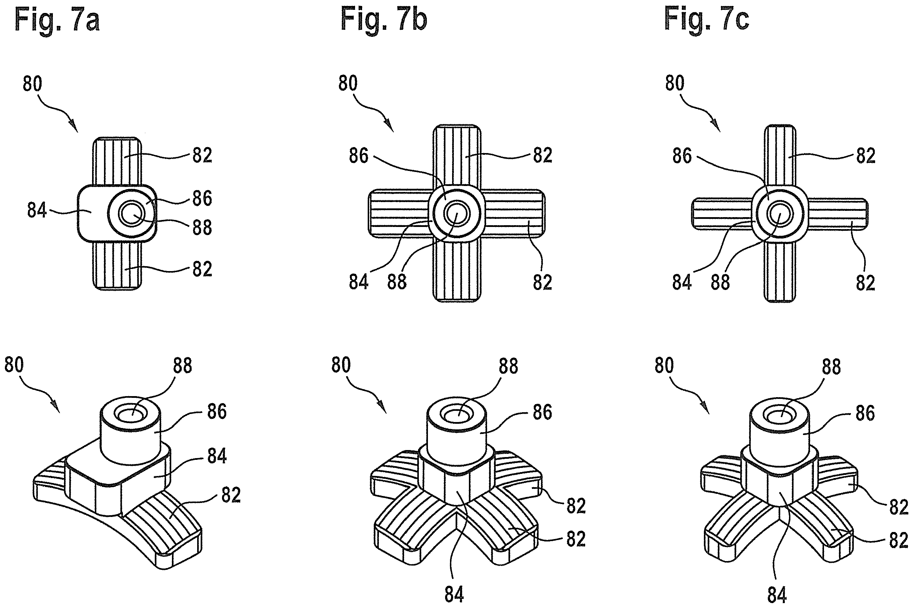

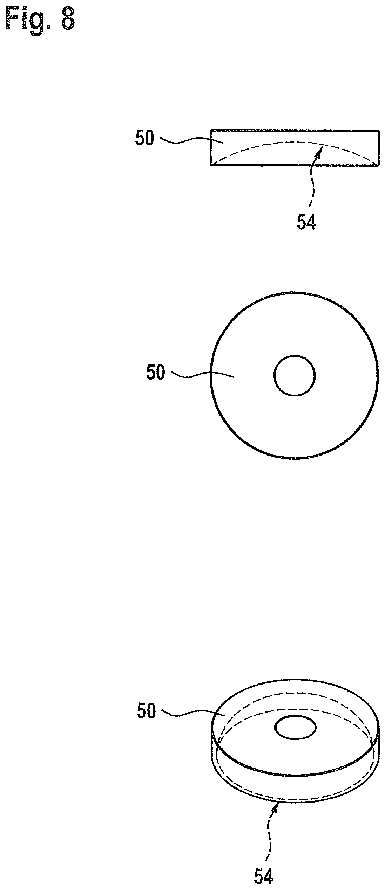

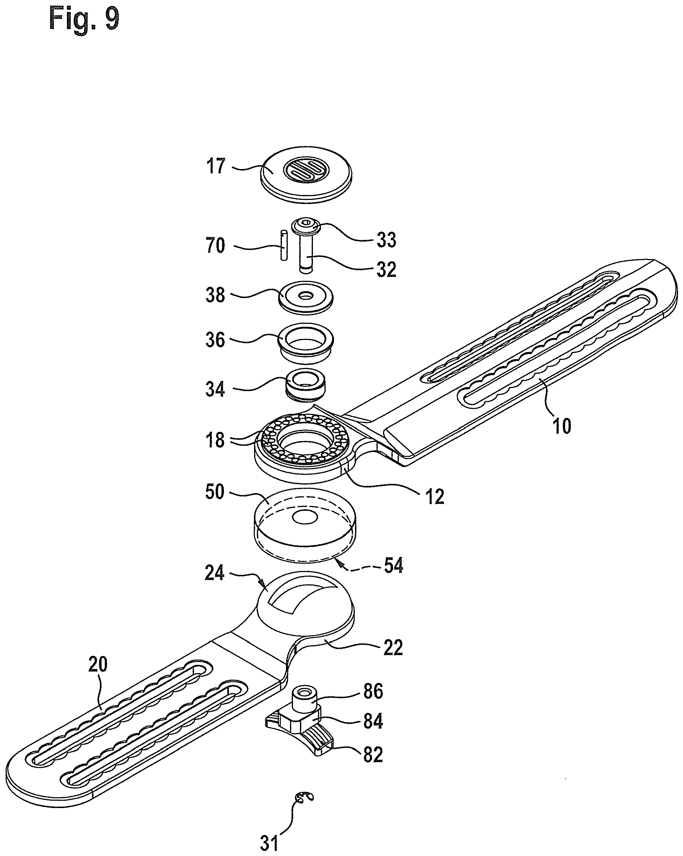

Figuren 7a, b, c zeigen jeweils verschieden alternative Ausgestaltungen eines Klemmsteins 80, jeweils in Draufsicht und Schrägansicht. Der Klemmstein 80 ist spezifisch für den Einsatz an einer kalottenförmigen Fläche gemäß Figuren 8 und 9 ausgebildet und ist in einen oberen zylindrischen Hals 86, welcher in die Lageranordnung 30 vorzugsweise spielfrei eingreifen kann, in einen Körper 84, welcher innerhalb des Fensters 26 des Kopfes 22 geführt werden kann, und in kreisbogenförmig gekrümmte Klemmabschnitte 82 unterteilt. Der Klemmstein 80 besitzt eine zentrale Bohrung 88, worin der Achsbolzen 32 der Lageranordnung 30 der erfindungsgemäßen Anordnung aufnehmbar ist. Die Bohrung 88 ist vorzugsweise als Schraubengewinde ausgeführt, worin der als Zylinderschraube ausgebildete Achsbolzen 32 einschraubbar ist, um insbesondere die Klemmung der gesamten Anordnung zu bewirken.
Figur 8 zeigt schematische Ansichten der Profilscheibe 50 mit einer unteren kalottenförmigen konkaven Ausnehmung 54, woran ein kalottenförmig gewölbter Kopf 22 gleiten kann.
Figur 9 zeigt eine Explosionsdarstellung einer anderen spezifischen Ausgestaltung, nach den Figuren 1, 2a, 2b und 5, welche alternativ zu der in der Figur 6 dargestellten Ausführung eine Lageranordnung mit einer Profilscheibe 50 mit konkaver kalottenförmiger Gleitfläche 54 aufweist, worin der Kopf 22 der zweiten Gelenkschiene 20 mit einer kalottenförmigen Gleitfläche 24 geführt und über den Klemmstein 80 dort festgeklemmt werden kann. Neben der reinen (uniaxialen) Kippbewegung der beiden Gelenkschenkel 10, 20 zueinander, wie sie die Ausführung gemäß Figur 6 ermöglicht, erlaubt die dargestellte alternative Ausführung eine Torsion der Gelenkschenkel 10, 20 zueinander, zusätzlich oder alternativ zu der Verkippung gegen die primäre Schwenkebene der Gelenkschiene.

## Patentansprüche

1. Gelenkschiene für eine Gelenkorthese mit zwei aneinander in einer zentralen Lageranordnung (30) schwenkbar gelagerten Gelenkschenkeln (10,20),
wobei der Kopf (12) des ersten Gelenkschenkels (10) die Lageranordnung (30) trägt und die Lageranordnung (30) mit dem Kopf (22) des zweiten Gelenkschenkels (20) kraftschlüssig verbindbar ist, um das Schwenkgelenk der beiden Gelenkschenkel (10,20) zu bilden,
wobei an der Lageranordnung (30) zu dem Kopf (22) des zweiten Gelenkschenkels (20) hin eine gekrümmte Gleitfläche (44,54) ausgebildet ist und der Kopf (22) des zweiten Gelenkschenkels (20) eine dazu formkorrespondierende Krümmung (24) aufweist, sodass der Kopf (22) des zweiten Gelenkschenkels (20) in der gekrümmten Gleitfläche (44) formschlüssig liegt und geführt ist und so ein Kippgelenk gebildet ist, worin der zweite Gelenkschenkel (20) zur Schwenkachse hin gegen den ersten Gelenkschenkel (10) verkippbar ist, **dadurch gekennzeichnet, dass**
der Kopf (22) des zweiten Gelenkschenkels (20) ein Fenster (26) aufweist, worin ein Klemmstein (60,80) geführt ist, der an die Lageranordnung (30) des ersten Gelenkschenkels (10) spannbar ist, um den Kopf (22) des zweiten Gelenkschenkels (20) in der gekrümmten Gleitfläche (44,54) kraftschlüssig zu fixieren, und dass der Klemmstein (60,80) Klemmschultern (62,82) aufweist, welche die Rückseite des Kopfes (22) hintergreifen, um den Kopf (22) an die Lageranordnung (30) des ersten Gelenkschenkels (10) zu klemmen.

2. Gelenkschiene nach Anspruch 1, wobei die Lageranordnung (30) an der Basis eine Profilscheibe (40,50) enthält, woran die gekrümmte Gleitfläche (44,54) ausgebildet ist.

3. Gelenkschiene nach Anspruch 1 oder 2, wobei die Klemmschultern (62,82) Abschnitte mit rauher oder gezahnter Oberfläche aufweisen, die mit einer entsprechenden Rastfläche an der Unterseite des Kopfes (22) in Eingriff kommen.

4. Gelenkschiene nach einem der Ansprüche 1 bis 3, wobei der Klemmstein (60,80) einen Hals (66,86) aufweist, der in die Lageranordnung (30) eingreift und dort geführt ist.

5. Gelenkschiene nach einem der Ansprüche 2 bis 4, wobei die Profilscheibe (40,50) eine Ausnehmung (46) aufweist, worin der Körper (64,84) des Klemmsteins (60,80) formschlüssig eingreifen kann, um den Klemmstein (60) gegen ein Verdrehen gegen die Gleitfläche (44,54) zu sichern.

6. Gelenkschiene nach Anspruch 5, wobei die das Fenster (26) des gewölbten Kopfes (22) so dimensioniert ist, dass der Körper (64) des Klemmsteins (60) dort formschlüssig eingreifen kann, um den Klemmstein (60) gegen ein Verdrehen gegen den Kopf (22) zu sichern.

7. Gelenkschiene nach einem der vorstehenden Ansprüche, wobei die Gleitfläche (44) und die Krümmung (24) des Kopfes (22) jeweils zylindermantelförmig ausgebildet sind und zusammen einen Axiallagerabschnitt bilden.

8. Gelenkschiene nach einem der Ansprüche 1 bis 5, wobei die Gleitfläche (54) und die Krümmung (24) des Kopfes (22) jeweils kalottenförmig ausgebildet sind und zusammen einen Kugelgelenkabschnitt bilden.

9. Gelenkschiene nach einem der Ansprüche 2 bis 8, wobei die Profilscheibe (40,50) eine Kulisse (48) aufweist, worin mindestens ein in dem Kopf (12) in Aufnahmen (18) geführter Limitierungsstift (70) eingreifen kann, um eine Schwenklimitierung zu bilden.

10. Gelenkorthese zur Stützung oder Korrektur eines Körpergelenks, enthaltend mindestens eine Gelenkschiene nach einem der vorstehenden Ansprüche.

## Claims

1. A joint brace for a joint orthosis comprising two articulated limbs (10, 20) pivotably mounted on each other in a central bearing arrangement (30),
wherein the head (12) of the first articulated limb (10) supports the bearing arrangement (30) and the bearing arrangement (30) can be connected by force-fit to the head (22) of the second articulated limb (20), in order to form the pivot joint of the two articulated limbs (10, 20),
wherein, on the bearing arrangement (30), a sliding surface (44, 54) curved toward the head (22) of the second articulated limb (20) is formed, and the head (22) of the second articulated limb (20) has a curvature (24) of matching shape, so that the head (22) of the second articulated limb (20) lies and is guided with positive-fit in the curved sliding surface (44), and in this manner a tilting joint is formed, wherein the second articulated limb (20) can be tilted toward the pivot axis against the first articulated limb (10), **characterized in that**
the head (22) of the second articulated limb (20) has a window (26), wherein a clamping block (60, 80) is guided, which can be clamped on the bearing arrangement (30) of the first articulated limb (10), in order to fix the head (22) of the second articulated limb (20) by force-fit in the curved sliding surface (44, 54), and the clamping block (60, 80) has clamping shoulders (62, 82), which engage behind the backside of the head (22), in order to clamp the head (22) on the bearing arrangement (30) of the first articulated limb (10).

2. The joint brace according to Claim 1, wherein the bearing arrangement (30) on the base contains a profile disk (40, 50), on which the curved sliding surface (44, 54) is formed.

3. The joint brace according to Claim 1 or 2, wherein the clamping shoulders (62, 82) have sections with a rough or toothed surface, which engage with a corresponding catch surface on the lower side of the head (22).

4. The joint brace according to any one of Claims 1 to 3, wherein the clamping block (60, 80) has a neck (66, 86) which engages in the bearing arrangement (30) and is guided there.

5. The joint brace according to any one of Claims 2 to 4, wherein the profile disk (40, 50) has a recess (46), wherein the body (64, 84) of the clamping block (60, 80) can engage with positive-fit, in order to prevent the clamping block (60) from being twisted against the sliding surface (44, 54).

6. The joint brace according to Claim 5, wherein the window (26) of the arched head (22) is dimensioned so that the body (64) of the clamping block (60) can engage with positive-fit there, in order to prevent the clamping block (60) from being twisted against the head (22).

7. The joint brace according to any one of the preceding claims, wherein the sliding surface (44) and the curvature (24) of the head (22) in each case are designed in the shape of a cylinder jacket and together form an axial bearing section.

8. The joint brace according to any one of Claims 1 to 5, wherein the sliding surface (54) and the curvature (24) of the head (22) in each case have a dome-shaped design and together form a ball joint section.

9. The joint brace according to any one of Claims 2 to 8, wherein the profile disk (40, 50) has a slotted link (48), wherein at least one limiting pin (70) guided in recesses (18) in the head (12) can engage, in order to form a pivot limitation.

10. A joint orthosis for the support or correction of a body joint, containing at least one joint brace according to any one of the preceding claims.

## Revendications

1. Attelle articulée pour une orthèse articulaire avec deux pieds articulés (10, 20) montés l'un sur l'autre de manière pivotante dans un assemblage de palier central (30),
la tête (12) du premier pied articulé (10) portant l'assemblage de palier (30), et l'assemblage de palier (30) étant reliable avec la tête (22) du second pied articulé (20) par friction pour former l'articulation pivotante des deux pieds articulés (10, 20),
dans laquelle une surface de glissement (44, 54) incurvée est formée sur l'assemblage de palier (30) vers la tête (22) du second pied articulé (20), et la tête (22) du second pied articulé (20) a une courbure en forme correspondante (24), de telle manière que la tête (22) du second pied articulé (20) reste dans la surface de glissement (44) incurvée en engagement positif et est guidée par celle-ci, ainsi formant une articulation basculante dans laquelle le second pied articulé (20) peut être basculer vers l'axe pivotant et contre le premier pied articulé (10), **caractérisée en ce que**
la tête (22) du second pied articulé (20) a une fenêtre (26) dans laquelle une pierre de serrage (60, 80) est guidée, la pierre étant serrable sur l'assemblage de palier (30) du premier pied articulé (10) pour fixer la tête (22) du second pied articulé (20) dans la surface de glissement (44, 54) incurvée par friction, et la pierre de serrage (60, 80) a des épaules de serrage (62, 82) qui viennent en prise sur le côté arrière de la tête (22) pour serrer la tête (22) sur l'assemblage de palier (30) du premier pied articulé (10).

2. Attelle articulée selon la revendication 1, dans laquelle l'assemblage de palier (30) contient au niveau de sa base un disque profilé (40, 50) sur lequel la surface de glissement (44, 54) incurvée est formée.

3. Attelle articulée selon la revendication 1 ou 2, dans laquelle les épaules de serrage (62, 82) présentent des sections avec une surface rugueuse ou dentelée qui viennent en prise avec une surface d'arrêt correspondante sur la face inférieure de la tête (22).

4. Attelle articulée selon l'une quelconque des revendications 1 à 3, dans laquelle la pierre de serrage (60, 80) présente un col (66, 86) qui vient en prise et est guidé dans l'assemblage de palier (30).

5. Attelle articulée selon l'une quelconque des revendications 2 à 4, dans laquelle le disque profilé (40, 50) présente un évidement (46) dans lequel le corps (64, 84) de la pierre de serrage (60, 80) peut s'installer en engagement positif, pour sécuriser la pierre de serrage (60) contre une rotation contre la surface de glissement (44, 54).

6. Attelle articulée selon la revendication 5, dans laquelle la fenêtre (26) de la tête courbe (22) est dimensionnée de telle manière que le corps (64) de la pierre de serrage (60) peut s'y installer en engagement positif pour bloquer la pierre de serrage (60) contre une rotation contre la tête (22).

7. Attelle articulée selon l'une quelconque des revendications précédentes, dans laquelle la surface de glissement (44) et la courbure (24) de la tête (22) sont respectivement configurées sous forme d'enveloppe cylindrique et ensemble forment une partie de palier axial.

8. Attelle articulée selon l'une quelconque des revendications 1 à 5, dans laquelle la surface de glissement (54) et la courbure (24) de la tête (22) sont respectivement configurées sous forme de calotte et ensemble forment une partie d'articulation sphérique.

9. Attelle articulée selon l'une quelconque des revendications 2 à 8, dans laquelle le disque profilé (40, 50) présente une coulisse (48) dans laquelle peut venir en prise au moins une goupille de limitation (70) guidée dans la tête (12) dans des logements (18) pour former une limitation de pivotement.

10. Orthèse articulaire pour supporter ou corriger une articulation de corps, comprenant au moins une attelle articulée selon l'une des revendications précédentes.
